# EUROPEAN PATENT APPLICATION

(11) **EP 3 578 167 A1**
(43) Date of publication of application: **11.12.2019**
(21) Application number: 18382385.5
(22) Date of filing: 04.06.2018
(51) Int. Cl.: A61K 9/00, A61K 47/02, A61K 47/14, A61K 47/24, A61K 47/44, A61K 31/4409, A61K 31/4439, A61K 31/4965

(54) **SUSPENSION FORMULATION FOR THE TREATMENT OF TUBERCULOSIS**

(71) Applicant: Universitat de Barcelona, 08028 Barcelona (ES)
(72) Inventor: SUÑÉ NEGRE, José María, 08028 Barcelona (ES); MERCADÉ FRUTOS, Débora, 08024 Barcelona (ES)

(57) **Abstract**

Kit of products for extemporaneous preparation of an oily suspension formulation comprising: (i) a dry powder composition of anti-tuberculosis effective amounts of the active pharmaceutical ingredients (APIs): isoniazid, rifampicin and pyrazinamide; and (ii) an oily liquid dispersing medium for reaching a predetermined volume of the suspension formulation, comprising: adequate amounts of the dispersing agents: ethoxylated hydrogenated castor oil, simethicone, and dichlorodimethylsilane-treated silica; adequate amounts of one or more flavoring agents; and the amount of medium-chain triglycerides, as oily base, that is necessary for reaching the predetermined volume of the suspension formulation; wherein (i) and (ii) are packaged in physically separated containers, arranged to be mixed extemporaneously to form the suspension formulation. The formulation is stable for more than three weeks under fridge conditions, and it is useful as a first-line treatment of tuberculosis in humans, particularly in children, with easy dose control, little effort, and good adherence.

## Description

### TECHNICAL FIELD

The present invention relates to the field of pharmaceuticals for the treatment of tuberculosis (TB) in humans, particularly in children.

### BACKGROUND ART

TB is an infectious disease usually caused by the bacterium *Mycobacterium tuberculosis.* TB may infect any part of the body, but most commonly occurs in the lungs (known as pulmonary tuberculosis). The urgency of the problem of TB in children, whose full scope is still not fully known, cannot be underestimated. According to World Health Organization (WHO) estimates, about 1 million children became ill with TB and 250 000 children died of TB in 2016 (cf. www.who.int/tb/areas-of-work/children/en).

The recommended treatment of new-onset pulmonary TB involves a combination of antibiotics containing isoniazid, rifampicin and pyrazinamide. Tablets containing 50 mg of isoniazid, 120 mg of rifampicin and 300 mg of pyrazinamide are available in many countries (e.g. in the EU member states and in the USA), and they are recommended in the initial intensive phase of the short-course treatment of pulmonary TB (referred to as 'first-line TB treatment' in the following) in adults. Each tablet contains isoniazid, pyrazinamide and rifampicin in such a ratio that the administration of 9-12 mg/kg rifampicin, 4-5 mg/kg isoniazid and 23-30 mg/kg pyrazinamide can be achieved by giving 3 tablets daily to adult patients weighing less than 40 kg, 4 tablets to patients weighing 40-49 kg, 5 tablets to patients weighing 50-64 kg, and 6 tablets to patients weighing 65 kg or more. However, it is considered that the ratio of the three antibiotics may not be appropriate in children; in fact, higher mg/kg doses of isoniazid are usually given in children than in adults. WHO and UNICEF advise against the use of adult fixed-dose combinations (FDCs) in children, which may lead to under- or over-dosing unfavorable treatment outcomes, and increase the likelihood of developing drug resistance.

Current practice for first-line TB treatment in children is far from being satisfactory. Sometimes broken adult tablets are used, with the concomitant problem of incorrect dosing and difficulty of swallowing. Sometimes, in hospital pharmacy services, aqueous suspension formulations of some of the active ingredients are prepared *ad hoc,* what involves considerable work and stability uncertainties. Besides, bad taste of tablets and ad *hoc* formulations create important problems of adherence.

In 2015 the WHO recommended the use of water-dispersible FDCs tablets for children containing 50 mg of isoniazid (as adult tablets on the market), 75 mg of rifampicin (instead of the 120 mg in adult tablets) and 150 mg of pyrazinamide (instead of the 300 mg in adult tablets). Recommended dosage of number of daily tablets was as follows: 1 (4-7 kg), 2 (8-11 kg), 3 (12-15 kg), and 4 (16-24 kg). For children above 25 kg adult dosages were recommended. According to WHO, these pediatric FDC tablets are obtainable from the Global Drug Facility (cf. www.stoptb.org/gdf); it is doubtful, however, that they will reach the pharmaceutical market. Most important is the problem of stability of rifampicin in multisuspensions containing also isoniazid and pyrazinamide. It has been observed that, although aqueous cosuspension of isoniazid and pyrazinamide are substantially stable, the addition of rifampicin to either isoniazid or pyrazinamide in cosuspension or together in multisuspension leads to a marked fall in the concentration of rifampicin, thus making inadvisable the dispensing of aqueous multisuspensions containing rifampicin, isoniazid and pyrazinamide (cf. H.I. Seifart et al., "Stability of isoniazid, rifampin and pyrazinamide in suspensions used for the treatment of tuberculosis in children", Pediatr. Infect. Dis. J. 1991, vol. 10, pp. 827-831).

From the above it is understood that there are no fully appropriate pharmaceutical form for first-line TB treatment in children.

### SUMMARY OF INVENTION

An aspect of the present invention relates to the provision of a kit of products for extemporaneous preparation of an oily suspension formulation comprising:
(i) a dry powder composition of anti-tuberculosis effective amounts of the active pharmaceutical ingredients (APIs): isoniazid, rifampicin and pyrazinamide; and
(ii) an oily liquid dispersing medium for reaching a predetermined volume of the suspension formulation, comprising:
   - adequate amounts of the dispersing agents: ethoxylated hydrogenated castor oil, simethicone, and dichlorodimethylsilane-treated silica;
   - adequate amounts of one or more flavoring agents to mask the bad taste of the suspension formulation; and
   - the amount of medium-chain triglycerides, as oily base, that is necessary for reaching the predetermined volume of the suspension formulation;
wherein (i) and (ii) are packaged in physically separated containers, arranged to be mixed extemporaneously to form the suspension formulation.

The oily base, which is the main ingredient of the oily liquid dispersing medium, is a colorless to slightly yellowish oily liquid, practically odorless and tasteless, that is formed by medium-chain triglycerides, this name being the one used in the United States Pharmacopeia - National Formulary. Medium-chain triglycerides are mainly mixtures of triesters of glycerin with octanoic acid and decanoic acids, that have the CAS-RN (Chemical Abstracts Service - Reference Number) 73398-61-5, and are referred to by the International Nomenclature of Cosmetic Ingredients (INCI) name caprylic/capric glycerides/triglycerides. The European Pharmacopoeia (Ph. Eur. 2005) describes medium-chain triglycerides as the fixed oil extracted from the hard, dried fraction of the endosperm of *Cocos nucifera L.* or from the dried endosperm of *Elaeis guineenis Jacq. ,* saying that they consist of a mixture of triglycerides of saturated fatty acids, mainly of octanoic acid and of decanoic acid, containing not less than 95% of saturated fatty acids.

Ethoxylated hydrogenated castor oil, simethicone and dichlorodimethylsilane-treated silica are here jointly referred to as 'dispersing agents', as they contribute -surely through different mechanisms- to the dispersion of the APIs in the oily liquid dispersing medium. Nevertheless, it is worth mentioning that these dispersing agents are sometimes considered to play other roles.

Ethoxylated hydrogenated castor oil (with CAS RN 61788-85-0, and referred to as PEG-40 hydrogenated castor oil by INCI) is obtained by reacting hydrogenated castor oil with ethylene oxide. It is considered to be both a dispersing agent and an emulsifying agent. Cremophor® RH 40 (BASF) is the specific ethoxylated hydrogenated castor oil used in Example 1.

Simethicone (CAS RN 8050-81-5), also known as polydimethylsiloxane-silicon dioxide mixture, is sometimes referred to as antifoaming agent or water-repelling agent. Dow Corning® Q7-2243 is the specific simethicone used in Example 1.

Dichlorodimethylsilane-treated silica (CAS RN 68611-44-9) is sometimes referred to as thickening and suspending agent. A particular dichlorodimethylsilane-treated silica, named dichlorodimethylsilane-treated fumed silica or hydrophobic fumed silica (CAS RN 60842-32-2, sold e.g. under the trade name "Aerosil® R 972") is used in Example 1.

Ingredient weight/volume percentage concentrations are here expressed as ingredient weight in g contained in 100 mL of oily suspension formulation. Thus, in a particular embodiments of the invention, the kit has the following weight/volume percentage concentrations of anti-tuberculosis APIs: 0.5-2.0 g/100mL of isoniazid; 0.7-5.0 g/100mL of rifampicin; and 1.0-12.0 g/100mL of pyrazinamide. In other particular embodiments, percentage concentrations are: 0.7-1.8 g/100mL of isoniazid; 1.0-4.0 g/100mL of rifampicin; and 2.0-10.0 g/100mL of pyrazinamide. In other particular embodiments, percentage concentrations are: 1.0-1.4 g/100mL of isoniazid; 1.5-3.0 g/100mL of rifampicin; and 2.5-8.0 g/100mL of pyrazinamide. In embodiments close to the one illustrated in Example 1, percentage concentrations are: 1.3 g/100mL of isoniazid; 2.0 g/100mL of rifampicin; and 3.3 g/mL of pyrazinamide.

In particular embodiments, the dispersing agents are used in the following percentage concentrations: 2.0-8.0 g/100mL of ethoxylated hydrogenated castor oil; 1.0-3.0 g/100mL of simethicone; and 0.5-2.0 g/100mL of dichlorodimethylsilane-treated silica. In other particular embodiments, percentage concentrations are: 4.0-6.0 g/100mL of ethoxylated hydrogenated castor oil; 1.5-2.5 g/100mL of simethicone; and 0.8-1.5 g/100mL of dichlorodimethylsilane-treated silica. In embodiments close to the one illustrated in Example 1, percentage concentrations are 5.0 g/100mL of ethoxylated hydrogenated castor oil; 2.0 g/100mL of simethicone; and 1.0 g/100mL of dichlorodimethylsilane-treated silica.

In order to mask the bad taste of the suspension formulations according to the present invention, making the formulations bearable to children, one or more flavoring agents are added. In particular embodiments the flavoring agents are sucralose, menthol and/or chocolate flavor. In embodiments close to the one illustrated by Example 1, the weight/volume percentage concentrations of the flavoring agents are 0.2-0.5 g/100mL of sucralose; 0.03-0.07 g/100mL of menthol; and 0.3-0.6 g/100mL of chocolate flavor.

Another aspect of the present invention relates to the oily suspension formulations extemporaneously prepared from the above-disclosed kits, for use in the treatment of TB in humans, particularly wherein humans are children. In other words, the invention relates to methods of treatment of a human -particularly a child- who is suffering from TB, comprising the administration of a therapeutically effective amount of the oily suspension formulations extemporaneously prepared from the above-disclosed kits.

Although the kits of the present invention may be used in the first-line TB treatment in adults, they are particularly useful in the treatment of children. Ratios of the three antibiotics recommended for children can be easily included in the kits, thus avoiding the need to use current adult fixed-dose combinations (FDCs), and the concomitant difficulties of swallowing and risks of under- or over-dosing.

Compared with the extemporaneous aqueous suspensions of the three antibiotics prepared from the WHO-recommended pediatric FDC tablets, that have to be freshly prepared every time for treating one or few children, the oily suspension formulation prepared from the kit of the present invention has the advantage of being stable for more than three weeks under fridge conditions, thus being useful for many more treatments with less effort. Besides, the formulation of the invention allows an easy dose control (e.g. by counting the number of previously-calibrated drops), and removes the problem of bad taste, something very important for the children adherence to the treatment.

Throughout the description and claims the word 'comprise' and variations of the word, are not intended to exclude other technical features, additives, components, or steps. Additional objects, advantages and features of the invention will become apparent to those skilled in the art upon examination of the description or may be learned by practice of the invention. The following examples are provided by way of illustration, and they are not intended to be limiting of the present invention.

### DESCRIPTION OF EMBODIMENTS

### Example 1: Ingredients and extemporaneous preparation of a suspension formulation of isoniazid, rifampicin and pyrazinamide for first-line TB treatment in children

1.33 g of isoniazid (from Acros Organics), 2.00 g of rifampicin (from Fagron Iberica), and 3.33 g of pyrazinamide (from Acros Organics) were weighed and mixed. The resulting mixture was sifted through a 100 µm sieve, and it was stored in a glass container provided of stopper, here referred to as 'the APIs container'.

In a beaker, here referred to as 'the excipients container', provided of mechanical stirring and controlled heating, 91 mL of medium-chain triglycerides were introduced and warmed at 70 °C. While keeping stirring and temperature, 1.00 g of Aerosil® R-792 (a particular dichlorodimethylsilane-treated fumed silica or hydrophobic fumed silica, from Evonik Industries) were added until total homogenization. After, 5.00 g of Cremophor® RH 40 (a PEG-40 hydrogenated castor oil, from BASF) were added, until total homogenization. The suspension was allowed to cool until room temperature. Then, 2.00 g of Dow Corning® Q7-2243 (simethicone) were added, while keeping stirring. Finally, 0.35 g of sucralose, 0.05 g of menthol and 0.45 g of chocolate flavor were added, until total homogenization.

An extemporaneous preparation of a suspension formulations was obtained by pouring the content of 'the excipients container' into 'the APIs container', then the later container was stopped, shaken manually until homogenization, and kept in a fridge at 5-6 °C (analogously as it will be done in medical practice).

### Example 2. Stability assay of the suspension formulation extemporaneously prepared, compared with respective suspension formulations of separated antibiotics, under fridge conditions

Equipment: HPLC system (Dionex Ultimate 3000), using a column from Phenomenex, Inc (Luna® 5 µm phenyl-hexyl 100A, LC column 150 x 4.6 mm).

Chromatographic conditions. The mobile phase contained 2% (v/v) methanol and 98% of a buffer solution having 0.05% of orthophosphoric acid and 0.05% of tetramethylammonium chloride. The pH of the mobile phase was approximately 3.5. The flow rate was 1.0 mL/min, the wavelength 254 nm, and the temperature 25 °C.

Preparation of standard solutions. Respective standard solutions of isoniazid (0.13 mg/mL), rifampicin (0.20 mg/mL), and pyrazinamide (0.33 mg/mL) were prepared fresh daily, using a mixture of methanol:buffer solution (34:66 v/v).

Preparation of assay solutions. 1 g of the suspension formulation obtained in Example 1 was diluted to 100 mL, by using a mixture of methanol:buffer solution (34:66 v/v).

Assay procedure and calculations. 20 µL of assay solution were injected into the HPLC system under the described conditions. For comparison, a similar volume of the respective standard solutions, containing the same concentration of the drug, were injected. Peak areas of the drug were directly related to the concentrations.

Stability assay. Stability assays were performed in order to obtain information about the degradation of the active pharmaceutical ingredients under fridge conditions (5-6 °C). Samples were taken at 0, 1, 2, 3, 6, 7, 8, 9, 10, 13, 14, 15, 16, 17, 20, 21, 22, and 23 days from preparation. In all cases degradation was absent, within experimental error (approx. 1%). However, at room conditions (25 ± 2 °C and 60 ± 5 % relative humidity), degradation of rifampicin was about 10% in 2 days. Thus, degradation percentages of the three API in the suspension formulation of the present invention, including the one of rifampicin (the most critical), are well below the accepted 5% of initial API contents (cf. ICH "Harmonized Tripartite Guideline. Stability Testing of New Drug Substances and Products Q1A(R2)", referred to by EMA as CPMP/ICH/2736/99, August 2003).

## Claims

1. Kit of products for extemporaneous preparation of an oily suspension formulation comprising:
(i) a dry powder composition of anti-tuberculosis effective amounts of the active pharmaceutical ingredients (APIs): isoniazid, rifampicin and pyrazinamide; and
(ii) an oily liquid dispersing medium for reaching a predetermined volume of the suspension formulation, comprising:
- adequate amounts of the dispersing agents: ethoxylated hydrogenated castor oil, simethicone, and dichlorodimethylsilane-treated silica;
- adequate amounts of one or more flavoring agents to mask the bad taste of the suspension formulation; and
- the amount of medium-chain triglycerides, as oily base, that is necessary for reaching the predetermined volume of the suspension formulation;
wherein (i) and (ii) are packaged in physically separated containers, arranged to be mixed extemporaneously to form the suspension formulation.

2. The kit according to claim 1, wherein, for a predetermined volume of the oily suspension formulation of 100 mL, the anti-tuberculosis APIs are in the following amounts:
0.5-2.0 g of isoniazid
0.7-5.0 g of rifampicin; and
1.0-12.0 g of pyrazinamide;

3. The kit according to claim 2, wherein the antituberculosis APIs are in the following amounts:
0.7-1.8 g of isoniazid;
1.0-4.0 g of rifampicin; and
2.0-10.0 g of pyrazinamide.

4. The kit according to claim 3, wherein the antituberculosis APIs are in the following amounts:
1.0-1.4 g of isoniazid;
1.5-3.0 g of rifampicin; and
2.5-8.0 g of pyrazinamide.

5. The kit according to claim 4, wherein the antituberculosis APIs are in the following amounts:
1.3 g of isoniazid;
2.0 g of rifampicin; and
3.3 g of pyrazinamide.

6. The kit according to any one of claims 2-5, wherein the dispersing agents are in the following amounts:
2.0-8.0 g of ethoxylated hydrogenated castor oil;
1.0-3.0 g of simethicone; and
0.5-2.0 g of dichlorodimethylsilane-treated silica.

7. The kit according to claim 6, wherein the dispersing agents are in the following amounts:
4.0-6.0 g of ethoxylated hydrogenated castor oil;
1.5-2.5 g of simethicone; and
0.8-1.5 g of dichlorodimethylsilane-treated silica.

8. The kit according to claim 7, wherein the dispersing agents are in the following amounts:
5.0 g of ethoxylated hydrogenated castor oil;
2.0 g of simethicone; and
1.0 g of dichlorodimethylsilane-treated silica.

9. The kit according to any one of claims 1-8, wherein the ethoxylated hydrogenated castor oil is PEG-40 hydrogenated castor oil, and the dichlorodimethylsilane-treated silica is dichlorodimethylsilane-treated fumed silica.

10. The kit according to any one of claims 1-9, wherein the one or more flavoring agents are sucralose, menthol, chocolate flavor, or mixtures thereof.

11. The kit according to claim 10, wherein the flavoring agents are in the following weight/volume percentage concentrations:
0.2-0.5 g/100mL of sucralose;
0.03-0.07 g/100mL of menthol; and
0.3-0.6 g/100mL of chocolate flavor.

12. An oily suspension formulation prepared from the kit as described in any one of claims 1-11, for use in the treatment of tuberculosis in humans.

13. The formulation for use according to claim 12, wherein humans are children.
